# EUROPÄISCHE PATENTSCHRIFT

(11) **EP 0 710 686 B1**
(45) Veröffentlichungstag und Bekanntmachung des Hinweises auf die Patenterteilung: **17.12.2003**
(21) Anmeldenummer: 95115587.8
(22) Anmeldetag: 04.10.1995
(51) Int. Cl.: C08G 63/672, C08G 63/91, C08G 18/42, C08J 11/24

(54) **Polyole und Verfahren zu deren Herstellung aus PET-Abfällen sowie Abfällen der PET-Production**
Polyols and process for their preparation from PET-waste or waste from the PET-production
Polyols et procédé pour leur préparation à partir de déchets de PET et de résidus de la production de PET

(30) Priorität: 17.10.1994 DE 4437043
(43) Veröffentlichungstag der Anmeldung: 08.05.1996
(73) Patentinhaber: ARTEVA TECHNOLOGIES S.à.r.l., 8001 Zürich (CH)
(72) Erfinder: Reck, Werner, Dipl.-Ing., D-03172 Guben (DE)
(74) Vertreter: Plate, Jürgen, Dr.

(56) Entgegenhaltungen:
- US-A- 4 469 824
- US-A- 4 720 571
- JOURNAL OF CELLULAR PLASTICS, Bd. 21, Nr. 5, September 1985, LANCASTER, PENNSYLVANIA, USA, Seiten 338-344, XP002009936 J.S. CANADAY ET AL.:
- CHEMICAL ABSTRACTS, vol. 95, no. 11, 14.September 1981 Columbus, Ohio, US; abstract no. 96496, XP002009937 & NIPPON KAGAKU KAISHI, Bd. 6, 1981, Seiten 974-9, A. UEJIMA:
- CELLULAR POLYMERS, Bd. 14, Nr. 1, 1.Januar 1995, Seiten 14-40, XP000500547 TERSAC G ET AL: "PROPRIETES PHYSICOCHIMIQUES DES POLYOLS OBTENUS PAR POLYESTERIFICATION DES PRODUITS DE GLYCOLYSE DU POLYETHYLENETEREPHTALATE ET SYNTHESES DE POLYURETHANES (POLYOLS BY POLYESTERIFICATION OF GLYCOLYSIS PRODUCTS OF POLYETHYLENETEREPHTHALATE, THEIR PHYSICOCHEMICAL PROPERTIES AND RESULTANT POLYURETHANE F"

## Beschreibung

Die Erfindung betrifft Polyole in Form bifunktioneller aromatischer Polyesteralkohole, die eine Kombination von relativ niedriger Hydroxylzahl (OHZ) und relativ niedermolekularem Oligomerenanteil aufweisen, sowie ein Verfahren zu deren Herstellung aus Abfällen aus Polyethylenterephthalat (PET) oder aus Abfällen der PET-Produktion.

Aromatische Polyesteralkohole sind wertvolle Substanzen, die in der Technik in großem Umfang, vorzugsweise als Teil der sogenannten A-Komponente für die Herstellung von Polyurethan-Hartschaum, eingesetzt werden. Sie bewirken eine Erhöhung der Flammfestigkeit und der Steifigkeit des Schaumes.
Für die Herstellung dieser wertvollen Verbindungen existieren verschiedene Verfahren, bei denen als Ausgangsmaterialien ausschließlich oder teilweise Primärrohstoffe eingesetzt werden.

In Verfahren zur Herstellung von bifunktionellen aromatischen Polyesteralkoholen, bei welchen als Ausgangsmaterialien ausschließlich Primärrohstoffe verwendet werden, kommt als Säurekomponente im wesentlichen Phthalsäureanhydrid (Anhydrid der Orthophthalsäure) in Verbindung mit Diethylenglykol (DEG) als zweiwertige alkoholische Komponente zum Einsatz.

Aus der Literatur sind auch verschiedene Vorschläge bekannt, Terephthalsäure aus Polyethylenterephalatabfällen als Säurekomponente für die Herstellung von Polyolen zu verwenden. Diese Verfahren basieren auf der Glykolyse der Polyesterabfälle und Umesterung mit DEG.
Die nach diesen Verfahren hergestellten Terephthalsäure-Polyesteralkohole unterscheiden sich in ihrem physikalischen Verhalten von Polyesteralkoholen auf Basis von Orthophthalsäure insbesondere hinsichtlich ihrer Lagerstabilität. So scheiden sich aus den zunächst flüssigen Terephthalsäure-Polyesteralkoholen, hergestellt durch Glykolyse und Umesterung von PET-Abfall mit DEG, sofern sie einen für eine Weiterverarbeitung in Betracht kommenden Bereich der Hydroxylzahl (OHZ) aufweisen, bereits nach wenigen Tagen Lagerzeit zunehmend Feststoffe ab, welche schließlich einen Übergang des Produktes in eine gatschartige Konsistenz verursachen.

Zur Umgehung dieses Nachteiles wurde eine Reihe von Verfahren zur Herstellung von Polyesteralkoholen vorgeschlagen, bei denen neben Terephthalsäure auf Basis von PET-Abfällen weitere aromatische oder aliphatische Dikarbonsäuren zum Einsatz kommen. So werden beispielsweise in den Druckschriften US-A-4608432, US-A-4546169 und EP-A-0152915 Polyolmischungen, hergestellt aus Phthalsäureanhydrid, PET-Abfällen und DEG beschrieben. In den Schriften US-A-4439550, EP-A-0248570 und DE-C-3435014 werden neben Phthalsäureanhydrid Adipinsäure, Glutarsäure, Bernsteinsäure und Isophthalsäure als alleinige oder kombinierte Zusatzkomponenten vorgeschlagen. Der Molanteil an derartigen zusätzlichen Dikarbonsäuren liegt dabei in der Regel bei 50 % und mehr, bezogen auf den Gesamtsäuregehalt.

Bei einem weiteren Vorschlag, lagerstabile Terephthalsäure-Polyesteralkohole zu erhalten, ist vorgesehen, neben DEG Glykole mit höherer Molmasse für den Glykolyse- und Umesterungsprozeß einzusetzen. Im US-A-4444919 wird ein PET-Glykolysat auf Basis, von DEG und Triethylenglykol beschrieben. Im US-A-4469824 wird, die Verwendung eines Gemisches von DEG und Dipropylenglykol vorgeschlagen.

Bekannt geworden sind auch Verfahren, die beide Wege kombiniert anwenden.

So sieht das USP 4720571 vor, eine Mischung von PET-Abfall und DMT-Produktionsrückständen (Gemisch aus vorwiegend Methylestern der Iso-, Orthound Terephthalsäure) mit mindestens zwei höheren Glykolen umzusetzen.

Alle diese Vorschläge haben den gravierenden Nachteil, daß neben den PET-Abfällen wertvolle und kostenaufwendige Primärrohstoffe eingesetzt werden müssen. Bei Verwendung aliphatischer Dikarbonsäuren oder Glykolen mit höherer Molmasse als DEG ergibt sich als weiterer Nachteil eine Reduzierung des Aromatengehaltes im Polyol und damit eine Verminderung der positiven Effekte, die sich aus dem Einsatz aromatischer Polyesteralkohole, z.B. bei der Polyurethanherstellung, ergeben.

Es ist bekannt, daß Polyesteralkohole hinsichtlich ihrer Molmasse kein einheitliches Produkt darstellen. Die Molmassen unterliegen in gleicher Weise wie bei den hochpolymeren Polyestern einer Verteilung, die durch Schulz und Flory mathematisch formuliert wurde. Aus dieser funktionellen Beziehung leitet sich ab, daß das Gleichgewicht mit steigender OHZ ständig weiter zu Gunsten des Gehaltes an freiem DEG und zu Lasten des Gehaltes an höheren Oligomeren verschoben wird.
Im konkreten Fall bedeutet dies, daß in der Kurve der Molmassenverteilung eines Polyesteralkohols auf Basis von Terephthalsäure und DEG, beispielsweise mit einer OHZ von 315 mg KOH/g, das Maximum beim monomeren Diester liegt, wobei ca 14 Gew.-% freies DEG und ein mit steigenden Polymerisationsgrad abnehmenden Anteil an Oligomeren bis hin zum Polymerisationsgrad 10 in der Gleichgewichtsmischung vorliegt. Ein derartiges Produkt ist nicht lagerstabil.

Als lagerstabil haben sich nur solche Glykolysate erwiesen, die eine OHZ von über 700 mg KOH/g aufweisen. Polyole mit derart hoher OHZ sind aber auf Grund der bestehenden Äquivalenzbeziehungen für die Umsetzung mit Diisocyanaten ungeeignet.

Aufgabe der vorliegenden Erfindung ist es, Polyole in Form von aromatischen Polyesteralkoholen zur Verfügung zu stellen, bei denen die Säurekomponente im wesentlichen aus Terephthalsäure aus PET-Abfällen oder Abfällen der PET-Produktion, und die Diolkomponente im wesentlichen aus DEG besteht und die trotz dieser Zusammensetzung über eine gute Lagerstabilität verfügen.

Diese Aufgabe wird erfindungsgemäß durch ein Verfahren gelöst, welches die Herstellung von Polyesteralkoholen ermöglicht, die sich in ihrer Molmasseverteilung wesentlich von den bei bisher bekannten Verfahren anfallenden Produkten unterscheiden. Während bekannte Produkte - wie oben ausgeführt - aufgrund der Gleichgewichtsreaktion eine Molmasseverteilung aufweisen, welche voll oder angenähert durch die Lage des chemischen Gleichgewichts bestimmt ist, die der jeweils eingestellten OHZ entspricht, enthalten die erfindungsgemäßen Produkte einen erheblich geringeren Anteil höhermolekularer Oligomere.

Ein Gegenstand der vorliegenden Erfindung ist somit ein Verfahren zur Herstellung von Polyolen in Form bifunktioneller aromatischer Polyesteralkohole aus PET-Abfällen sowie Abfällen der PET-Produktion, das dadurch gekennzeichnet ist, daß
- die Abfälle in Diethylenglykol gelöst werden,
   wobei das Mengenverhältnis von Abfällen zu Diethylenglykol so gewählt wird, daß der nach der anschließenden Umesterung erhaltene Polyesteralkohol eine Hydroxylzahl größer 700 mg KOH/g aufweist,
- die in DEG gelösten und erforderlichen- oder gewünschtenfalls von Feststoffen befreiten Abfälle unter Einsatz bekannter Umesterungskatalysatoren umgeestert werden, wobei das hierbei freigesetzte Ethylenglykol zumindest teilweise abdestilliert wird,
- und daß aus dem im Gleichgewicht befindlichen Umesterungsprodukt bei einer Temperatur von unter 140 °C freies DEG so weit abdestilliert wird, daß ein Polyesteralkohol mit einer OHZ von 260 - 500 mg KOH/g erhalten wird.

Das erfindungsgemäße Verfahren beruht auf der überraschenden Erkenntnis, daß das bei höheren Hydroxylzahlen, beispielsweise bei einer OHZ von > 700 mg KOH/g, sich bezüglich des Verhältnisses zwischen monomerem Diester und Oligomeren einstellende Gleichgewicht erhalten bleibt, wenn das freie DEG bei einer Temperatur von unter 140 °C, vorzugsweise von max. 130 °C, destillativ abgetrennt und damit die OHZ auf den gewünschten Wert abgesenkt wird. Die Abtrennung des DEG bei dieser Temperatur ist erfindungsgemäß möglich, wenn der Prozeß bei einem Druck kleiner 2 hPa, vorzugsweise kleiner 1 hPa, durchgeführt wird. Auf diese Weise werden Polyesteralkohole erhalten, welche je nach Vollständigkeit der DEG-Abtrennung gezielt auf OHZ-Werte von 260 - 500 mg KOH/g eingestellt werden können. Die erhaltenen Produkte zeigen auch nach längerer Lagerung keine Abscheidung von Feststoffen.

Mit Hilfe der HPLC unter Anwendung von Tetrahydrofuran/Wasser als Eluent, wurden die eingetretenen Veränderungen in der Molmassenverteilung sichtbar nachgewiesen. Während die Verteilungskurve bei einem im Gleichgewicht befindlichen Produkt der OHZ 315 mg KOH/g bis zu Oligomeren mit einem Polymerisationsgrad (Pₙ) von 10 reichte, fällt sie bei dem erfindungsgemäßen Produkt bereits bei Oligomeren mit einem Polymerisationsgrad von über 6 praktisch auf Null, zumindest aber auf außerordentlich geringe Werte ab. Der sich hieraus für die erfindungsgemäßen Polyole ergebende Anteil von Oligomeren mit einem Polymerisationsgrad oberhalb 6, bezogen auf den Gesamtanteil von Oligomeren, dürfte in der Regel unter 1 Gew.-%, vorzugsweise unter 0,5 Gew.-% liegen.

Aus den Chromatogrammen der HPLC - Analyse eines typischen erfindungsgemäßen Polyols und eines herkömmlich hergestellten Polyols gleicher OHZ ergeben sich beispielsweise die aus der folgenden Gegenüberstellung ersichtlichen Werte für die Oligomerenanteile, bezogen auf den Gesamtanteil der Oligomeren (d.h. ohne monomeren Diester):

| Olgomerenanteile, bezogen auf Gesamtanteil der Oligomeren. | | |
|---|---|---|
| Polymerisationsgrad Pₙ des Oligomeren | Herkömmlich hergestelltes Polyol | Erfindungsgemäßes Polyol |
| Pₙ = 6 | 5,6 Gew.-% | 0,9 Gew.-% |
| Pₙ > 6 | 7,0 Gew.-% | nicht nachweisbar |

Erwärmt man das erfindungsgemäße Produkt wieder, so wird bei Temperaturen über 140 °C ein Ansteigen des DEG-Cehaltes und damit eine allmähliche Rückstellung des für die OHZ zutreffenden Gleichgewichtes festgestellt. Bei einer Temperatur von 230 °C stellt sich das volle Gleichgewicht binnen kurzer Zeit wieder ein. Die Produkteigenschaften entsprechen dann denen eines nach bekannten Verfahren hergestellten Terephthalsäure-Polyesteralkohols.

Das erfindungsgemäße Verfahren wird in der Form durchgeführt, daß die Abfälle zunächst in einem vorteilhafterweise mit Rühreinrichtung versehenen Reaktor unter Normaldruck und Stickstoffbeschleierung gelöst werden. Für hochpolymeres Material sind dafür Temperaturen bis 250 °C zweckmäßig, für niedermolekulare Destillationsrückstände sind Temperaturen bis 205 °C ausreichend. Die DEG-Menge ist so zu bemessen, daß nach dem späteren Umesterungsprozeß eine OHZ von größer 700 mg KOH/g gesichert erreicht wird. Hierzu ist je nach apparativen Bedingungen ein Molverhältnis von PET (bezogen auf die Molekulareinheitsmasse von 192 g/mol) zu DEG von 1 : 5 bis 1: 10, vorzugsweise von 1 : 6,6 bis 8,0 erforderlich.

Es kann vorteilhaft sein, den Löseprozeß nur mit einem Teil der notwendigen DEG-Menge durchzuführen und den Rest erst unmittelbar vor der Umesterungsstufe zu ergänzen. Dies trifft insbesondere für in der Zusammensetzung stark schwankende Destillationsrückstände der PET-Produktion zu, wo erst über die Bestimmung der Verseifungszahl und daraus abgeleitet des Terephthalsäuregehaltes nach dem Löseprozeß die erforderliche Restmenge an DEG ermittelt werden kann.

Falls erforderlich und gewünscht, werden die Abfälle nach vollständiger Lösung filtriert, wobei die Filtrationsbedingungen vom Grad der Verunreinigung abhängig sind. Bei TiO₂- haltigen Abfällen sowie bei Destillationsrückständen macht sich zur Erzielung eines klaren Filtrates der Einsatz von besonderen Filtermitteln erforderlich. Bewährt hat sich dafür beispielsweise eine Mischung aus gleichen Teilen von Zellstoffschnitzeln, kristalliner Mikrocellulose und gereinigtem Kieselgur als Filterschicht.

Vom Filtrat wird anschließend in einem Glykolyse- und Umesterungsprozeß das enthaltene Ethylenglykol (EG) freigesetzt und durch Destillation abgetrennt. Die weitgehende Entfernung des EG ist zweckmäßig, um Produkte niedriger Viskosität zu erhalten und die Abscheidung von Feststoffen gesichert zu vermeiden. Angestrebt wird ein EG-Gehalt von max. 10 %, vorteilhafterweise von max. 5 im Endprodukt. Für den zügigen Ablauf des Umesterungsprozesses sind bekannte Katalysatoren einzusetzen, beispielsweise Tetrabutyltitanat, Kobaltacetat, Manganacetat oder Zinkoxid.

Der Glykolyse- und Umesterungsprozeß kann beispielsweise in einem beheizbaren Rührreaktor durchgeführt werden, welcher mit einer Destillationskolonne ausgerüstet ist. Die Kolonne soll über eine ausreichende Trennwirkung verfügen, um den DEG-Gehalt im abdestillierenden EG möglichst gering zu halten. Für die Prozeßführung ist ein Teilvakuum erforderlich, dessen Höhe vom Strömungswiderstand der Kolonne mitbestimmt wird und zweckmäßigerweise so einzustellen ist, daß in der letzten Phase des Umesterungsprozesses eine Produkttemperatur von über 210°C, vorzugsweise über 225°C, beispielweise von 230 - 235 °C erreicht wird. Chromatographische Untersuchungen haben ergeben, daß erst bei dieser Temperatur der Anteil an Oligomeren innnerhalb einer technisch vorteilhaften Reaktionszeit bis auf den Gleichgewichtszustand abgesenkt ist. Je nach apparativer Bedingung kann der dazu erforderliche Druck in einem Bereich von 500 - 750 hPa liegen.

Nach Abschluß der EG-Abtrennung wird der Reaktorinhalt bis auf unter 140 °C gekühlt und unter Umgehung der Kolonne das DEG bei einem Restdruck von beispielsweise 1 hPa sowie einer Produkttemperatur von unter 140 °C, vorzugsweise von maximal 130°C so weit abdestilliert, bis die für das Produkt gewünschte OHZ erreicht ist. Diese kann wahlweise in einem Bereich zwischen 260 - 500 mg KOH/g liegen. Danach wird das Produkt ausgetragen.

Das erfindungsgemäße Verfahren ist anwendbar für PET-Abfälle, beispielsweise von Flaschen, Folien, Bändern, Filmmaterial und Fasern sowie für Abfälle der PET-Produktion, beispielsweise Destillationsrückstände der Brüdenaufbereitung, Gießabfälle, Rückstände aus der Chips-Entstaubung und Fehlchargen. Es hat den Vorteil einer höheren Wirtschaftlichkeit, da außer dem Abfallmaterial als Rohstoff nur DEG, welches als Nebenprodukt der EG-Herstellung ein Billigdiol darstellt, zum Einsatz gelangt.
Ein weiterer Vorteil des erfindungsgemäßen Verfahrens besteht in der niedrigeren Viskosität der danach hergestellten Polyesteralkohole gegenüber Polyesteralkoholen gleicher OHZ, die nach bekannten Verfahren hergestellt wurden.

Ein weiterer Gegenstand dieser Erfindung ist das nach dem oben beschriebenen erfindunggemäßen Verfahren herstellbare lagerfähige Polyol in Form eines bifunktionellen aromatischen Polyesteralkohols.
Dieses erfindungsgemäße Produkt ist dadurch gekennzeichnet, daß seine OHZ innerhalb eines Bereiches von 260 - 500 mg KOH/g liegt, und es einen Anteil von höheren Oligomeren enthält, der gegenüber dem Gleichgewichtsanteil, der der jeweiligen OHZ entspricht, herabgesetzt ist.
Bevorzugt sind solche erfindungsgemäßen Polyole, die, bezogen auf den Gesamt-Oligomerenanteil, nicht mehr als 1 Gew.-%, vorzugsweise nicht mehr als 0,5 Gew.-% von Oligomeren mit einem Polymerisationsgrad größer 6 enthalten, insbesondere solche, die praktisch frei sind von Oligomeren mit einem Polymerisationsgrad größer 6.
Weiterhin sind solche erfindungsgemäßen, lagerfähigen Polyole bevorzugt, deren Gehalt an freiem und gebundenem EG max. 10 Gew.-% beträgt.

Die erfindungsgemäßen Polyole eignen sich für alle technischen Anwendungen, bei denen der Einsatz von Polyolen mit relativ niedriger OHZ, die einen sehr geringen Anteil höhermolekularer Ologomere aufweisen, erforderlich oder erwünscht ist, vorzugsweise beispielsweise als Bestandteil der A-Komponente bei der Herstellung von Polyurethanen, insbesondere von Polyurethanschäumen. Die folgenden Ausführungsbeispiele veranschaulichen besonders vorteilhafte Ausführungsformen des erfindungsgemäßen Verfahrens.

### Beispiel 1

1000 g PET-Flaschenabfälle wurden in einem 6 l fassenden Reaktionsgefäß in 3700 g DEG bei einer Temperatur bis 240 °C unter Rühren und Stickstoffbeschleierung gelöst. Nach Abschluß des Löseprozesses wurde das Produkt auf 180 °C abgekühlt und durch Filtration über eine mit Filterpapier ausgelegte Nutsche von den als Feststoffe enthaltenen Verunreinigungen getrennt.

Das Filtrat wurde in einem gleichfalls 6 l fassenden beheizbaren Rührreaktor mit aufgesetzter Kolonne überführt und nach Zugabe von 200 mg Tetrabutyltitanat bei einem Druck von 680 hPa erwärmt. Die Kolonne war mit 10 Glockenböden und beheizbarem Außenmantel ausgerüstet. Nach Erreichen einer Temperatur von 225 °C begann in Verbindung mit dem sich vollziehenden Glykolyseund Umesterungsprozeß das Abdestillieren von EG. Durch Einstellung der Kolonnenmantel-Temperatur auf 186 °C und Regelung der Kopftemperatur durch Variieren des Rücklaufverhältnisses auf gleichfalls 186 °C konnte das abdestillierende EG weitgehend von DEG abgetrennt werden. Die Produkttemperatur erhöhte sich mit der Menge des abdestillierten EG und betrug am Prozeßende 235 °C bei einer auf 175 °C abgesunkenen Kopftemperatur. Das Umesterungsprodukt wies eine OHZ von 728 mg KOH/g auf.

Anschließend wurde das Produkt auf 130 °C abgekühlt und durch allmähliche Erhöhung des Vakuums das freie DEG unter Umgehung der Kolonne abdestilliert. Der Druck am Ende des Destillationsprozesses betrug 0,2 hPa, die Temperatur des Produktes 130 °C.

Es wurden 1770 g hellgelbes Polyol mit einer Säurezahl von 0,9 mg KOH/g, einer OHZ von 325 mg KOH/g und einer Viskosität bei 25 °C von 2100 mPa•s erhalten. Aus dem Produkt schieden sich bei der Lagerung keine Feststoffe ab.

### Beispiel 2

1808 g Destillationsrückstände aus der Brüdenaufbereitung der PET-Produktion wurden in einem 6 l fassenden Reaktionsgefäß in gleicher Menge an DEG bei einer Temperatur von 205 °C unter Rühren und Stickstoffbeschleierung gelöst. Die stark getrübte Lösung wurde anschließend auf 180 °C abgekühlt und über eine Nutsche mit Filterschicht, bestehend aus einem Gemisch von je 25 g Zellstoffschnitzeln, kristalliner Mikrocellulose und gereinigtem Kieselgur filtriert.

Das in einer Menge von 3597 g erhaltene klare Filtrat wies eine Verseifungszahl von 228,5 mg KOH/g entsprechend einem Gehalt an Terephthalsäure von 33,9 % auf. Zur Erreichung der gewünschten OHZ von größer 700 mg KOH/g nach Abschluß des Umesterungsprozesses wurden dem Filtrat unter Berücksichtigung der bereits in den Destillationsrückständen enthaltenen DEG-Menge 2795 g DEG und als Umesterungskatalysator 360 mg Tetrabutyltitanat zugesetzt.

Das Gemisch wurde analog Beispiel 1 weiter behandelt, wobei das Umesterungsprodukt eine OHZ von 709 mg KOH/g aufwies.

Es wurden 2274 g hellbraunes Polyol mit einer Säurezahl von 1,1 mg KOH/g, einer OHZ von 305 mg KOH/g und einer Viskosität bei 25 °C von 3500 mPa•s erhalten. Aus dem Produkt schieden sich bei der Lagerung keine Feststoffe aus.

## Patentansprüche

1. Verfahren zur Herstellung von Polyolen in Form bifunktioneller aromatischer Polyesteralkohole aus PET-Abfällen sowie Abfällen der PET-Produktion, **dadurch gekennzeichnet, daß**
- die Abfälle in Diethylenglykol (DEG) gelöst werden,
wobei ein Molverhältnis von PET (bezogen auf die Molekulareinheitsmasse von 192 g/mol) zu DEG von 1:5 bis 1: 10 gewählt wird, so daß der nach der anschließenden Umesterung erhaltene Polyesteralkohol eine Hydroxylzahl (OHZ) größer 700 mg KOH/g aufweist,
- die in DEG gelösten und erforderlichen- oder gewünschtenfalls von Feststoffen befreiten Abfälle unter Einsatz bekannter Umesterungskatalysatoren umgeestert werden, wobei das hierbei freigesetzte Ethylenglykol (EG) zumindest teilweise abdestilliert wird,
- und daß aus dem im Gleichgewicht befindlichen Umesterungsprodukt bei einer Temperatur von unter 140 °C freies DEG so weit abdestilliert wird, daß ein Polyesteralkohol mit einer OHZ von 260 - 500 mg KOH/g erhalten wird.

2. Verfahren nach Anspruch 1, **dadurch gekennzeichnet, daß** die Lösung der Abfälle in DEG bei einer vom Polymerisationsgrad der Abfälle abhängigen Mindesttemperatur von 200 - 250 °C erfolgt.

3. Verfahren nach mindestens einem der Ansprüche 1 und 2, **dadurch gekennzeichnet, daß** der Löseprozeß nur mit einer Teilmenge des erforderlichen DEG-Anteiles erfolgt und die Restmenge nach analytischer Charakterisierung des Produktes vor dem Umesterungsprozeß zugegeben wird.

4. Verfahren nach mindestens einem der Ansprüche 1 bis 3, **dadurch gekennzeichnet, daß** die in DEG gelösten Abfälle zur Abtrennung enthaltener Feststoffe in Form von Schwebstoffen oder Suspensionen erforderlichenfalls unter Einsatz von Filtermitteln filtriert werden.

5. Verfahren nach mindestens einem der Ansprüche 1 bis 3, **dadurch gekennzeichnet, daß** bei der Umesterung das EG soweit abdestilliert wird, daß das Endprodukt einen Gehalt von max. 10 Gew.-%, vorzugsweise von max. 5 Gew.-% EG in freier und gebundener Form aufweist.

6. Verfahren nach mindestens einem der Ansprüche 1 bis 5, **dadurch gekennzeichnet, daß** die Umesterung bei einem Druck von 500 - 700 hPa und einer Produkttemperatur bis maximal 235 °C erfolgt.

7. Verfahren nach mindestens einem der Ansprüche 1 bis 6, **dadurch gekennzeichnet, daß** aus dem im Gleichgewicht befindlichen Umesterungsprodukt freies DEG bei einer Temperatur von max. 130 °C abdestilliert wird.

8. Lagerfähiges Polyol in Form eines bifunktionellen aromatischen Polyesteralkohols, **dadurch gekennzeichnet, daß** es nach einem Verfahren gemäβ einem oder mehreren des Anprüche 1 bis 7 erhältlich ist.

9. Lagerfähiges Polyol gemäß Anspruch 8, **dadurch gekennzeichnet, daß** es, bezogen auf den Gesamt-Oligomerenanteil, nicht mehr als 1 Gew.-%, vorzugsweise nicht mehr als 0.5 Gew.-% von Oligomeren mit einem Polymerisationsgrad größer 6 enthält.

10. Lagerfähiges Polyol gemäß Anspruch 8 oder 9, **dadurch gekennzeichnet, daß** es praktisch frei ist von Oligomeren mit einem Polymerisationsgrad größer 6.

11. Lagerfähiges Polyol gemäß mindestens einem der Ansprüche 8 bis 10, **dadurch gekennzeichnet, daß** sein Gehalt an freiem und gebundenem EG max. 10 Gew.-% beträgt.

12. Verwendung des Polyols gemäß eineim oder mehreren der Ansprüche 8 bis 11 bei der Herstellung von Polyurethanen, insbesondere von Polyurethan-Schäumen.

## Claims

1. A process for preparing polyols in the form of bifunctional aromatic polyester alcohols from PET wastes and wastes from PET production, which comprises
- dissolving the wastes in diethylene glycol (DEG) in a molar ratio of from 1:5 to 1:10 for PET (based on the molecular unit mass of 192 g/mol) to DEG, so that the polyester alcohol obtained after the subsequent transesterification has a hydroxyl number (OHN) greater than 700 mg KOH/g,
- transesterifying the wastes dissolved in DEG and, if required or if desired, freed of solids, using known transesterification catalysts, with the ethylene glycol (EG) liberated in the process being at least partially distilled off,
- and distilling off free DEG at a temperature of below 140°C from the transesterification product at equilibrium until a polyester alcohol having an OHN of 260-500 mg KOH/g is obtained.

2. The process as claimed in claim 1, wherein the dissolution of the wastes in DEG is carried out at a minimum temperature of 200-250°C depending on the degree of polymerization of the wastes.

3. The process as claimed in claim 1 or 2, wherein the dissolution process is carried out using only a part of the amount of DEG required and the remaining amount is added before the transesterification process after analytical characterization of the product.

4. The process as claimed in at least one of claims 1 to 3, wherein the wastes dissolved in DEG are filtered to remove solids present in the form of suspended materials or suspensions, if necessary using filter aids.

5. The process as claimed in at least one of claims 1 to 3, wherein during the transesterification the EG is distilled off to such an extent that the final product has a content of at most 10% by weight, preferably at most 5% by weight, of EG in free and bound forms.

6. The process as claimed in at least one of claims 1 to 5, wherein the transesterification is carried out at a pressure of 500-700 hPa and a product temperature of up to a maximum of 235°C.

7. The process as claimed in at least one of claims 1 to 6, wherein free DEG is distilled off at a temperature of at most 130°C from the transesterification product at equilibrium.

8. A storage-stable polyol in the form of a bifunctional aromatic polyester alcohol which is obtainable by a process as claimed in one or more of claims 1 to 7.

9. A storage-stable polyol as claimed in claim 8, containing, based on the total oligomer content, not more than: 1% by weight, preferably not more than 0.5% by weight, of oligomers having a degree of polymerization of greater than 6.

10. A storage-stable polyol as claimed in claim 8 or 9, which is virtually free of oligomers having a degree of polymerization of greater than 6.

11. A storage-stable polyol as claimed in at least one of claims 8 to 10, having a content of free and bound EG of at most 10% by weight.

12. Use of the polyol as claimed in one or more of claims 8 to 11 in the production of polyurethanes, in particular of polyurethane foams.

## Revendications

1. Procédé pour la fabrication de polyols sous la forme de polyalcools d'ester aromatiques bifonctionnels à partir de déchets de PET ainsi qu'à partir de déchets de la production de PET, **caractérisé en ce que**
- les déchets sont dissous dans du diéthylèneglycol (DEG), en sélectionnant un rapport molaire PET (sur la base de la masse unitaire moléculaire de 192 g/mole) / DEG de 1:5 à 1:10, de façon à ce que le polyalcool d'ester obtenu après la transestérification finale présente un indice d'hydroxyle (OHZ) supérieur à 700 mg KOH/g..
- les déchets dissous dans le DEG et le cas échéant ou au besoin débarrassés des substances solides sont transestérifiés en utilisant des catalyseurs de transestérification connus, l'éhtylèneglycol (EG) ainsi libéré étant au moins partiellement extrait par distillation,
- et **en ce que** le DEG libre est extrait par distillation du produit de transestérification se trouvant à l'état stable, à une température inférieure à 140° C, jusqu'à ce que soit obtenu un polyalcool d'ester présentant un indice d'hydroxyle de 260 à 500 mg KOH/g

2. Procédé selon la revendication 1, **caractérisé en ce que** la dissolution des déchets dans le DEG se fait à une température minimum de 200 à 250° C fonction du degré de polymérisation des déchets.

3. Procédé selon au moins une des revendications 1 et 2, **caractérisé en ce que** le processus de dissolution ne se fait qu'avec une quantité partielle de la part de DEG nécessaire, et que la quantité résiduelle est ajoutée après caractérisation analytique du produit avant le processus de transestérification.

4. Procédé selon au moins une des revendications 1 à 3, **caractérisé en ce que** les déchets dissous dans le DEG sont si nécessaire filtrés en utilisant des moyens de filtration pour séparation des matières solides qu'ils contiennent sous la forme de sédiments ou matières en suspension.

5. Procédé selon au moins une des revendications 1 à 3, **caractérisé en ce que** l'EG est extrait par distillation pendant la transestérification jusqu'au point où le produit fini présente une teneur en EG sous forme libre et liée d'un maximum de 10 % en poids, de préférence d'un maximum de 5 % en poids.

6. Procédé selon au moins une des revendications 1 à 5, **caractérisé en ce que** la transestérification se fait à une pression de 500 à 700 hPa, et à une température du produit de 235° C maximum.

7. Procédé selon au moins une des revendications 1 à 6, **caractérisé en ce que** le DEG libre est extrait par distillation du produit de transestérification se trouvant à l'état stable à une température maximum de 130° C.

8. Polyol stockable sous la forme de polyalcool d'ester aromatique bifonctionnel, **caractérisé en ce qu'**il peut être obtenu selon un procédé selon une ou plusieurs des revendications 1 à 7.

9. Polyol stockable selon la revendication 8, **caractérisé en ce qu'**il ne contient, sur la base de la part totale d'oligomères, pas plus de 1 % en poids, de préférence pas plus de 0,5 % en poids, d'oligomères présentant un degré de polymérisation supérieur à 6.

10. Polyol stockable selon les revendications 8 ou 9, **caractérisé en ce qu'**il est pratiquement exempt d'oligomères présentant un degré de polymérisation supérieur à 6.

11. Polyol stockable selon au moins une des revendications 8 à 10, **caractérisé en ce que** sa teneur en EG libre et lié s'élève à un maximum de 10 % en poids.

12. Utilisation du polyol selon une ou plusieurs des revendications 8 à 11 pour la fabrication de polyuréthanes, en particulier de mousses de polyuréthane.
